(19) Europäisches Patentamt
European Patent Office
Office européen des brevets

(11) Veröffentlichungsnummer: **0 132 690**
**B1**

(12) # EUROPÄISCHE PATENTSCHRIFT

(45) Veröffentlichungstag der Patentschrift: **01.02.89**

(51) Int. Cl.⁴: **C 08 K 5/00**, A 61 L 27/00, A 61 L 29/00

(21) Anmeldenummer: **84108048.4**

(22) Anmeldetag: **10.07.84**

(54) **Antiphlogistika enthaltende thermoplastische Kunststoffe.**

(30) Priorität: **21.07.83 DE 3326209**
**31.12.83 DE 3347660**

(43) Veröffentlichungstag der Anmeldung: **13.02.85 Patentblatt 85/07**

(45) Bekanntmachung des Hinweises auf die Patenterteilung: **01.02.89 Patentblatt 89/05**

(84) Benannte Vertragsstaaten: **AT BE CH DE FR GB IT LI LU NL SE**

(56) Entgegenhaltungen:
**EP-A- 0 016 652**
**FR-A- 2 372 623**
**GB-A- 2 086 224**

**Die Akte enthält technische Angaben, die nach dem Eingang der Anmeldung eingereicht wurden und die nicht in dieser Patentschrift enthalten sind.**

(73) Patentinhaber: **Troponwerke GmbH & Co. KG**
**Berliner Strasse 156**
**D-5000 Köln 80 (DE)**

(72) Erfinder: **Dell, Hans-Dieter, Dr.**
**Kalmüntener Strasse 5**
**D-5060 Bergisch-Gladbach 2 (DE)**
Erfinder: **Pelster, Eberhard, Dr.**
**Pleisufer 6a**
**D-5205 St. Augustin 1 (DE)**
Erfinder: **Wirzbach, Eberhard**
**Wittekindstrasse 6**
**D-4715 Ascheberg (DE)**
Erfinder: **Burgdörfer, Hans-Heribert, Dr.**
**Goffineweg 49**
**D-5000 Köln 80 (DE)**
Erfinder: **Ascherl, Rudolf, Dr.**
**Rathausstrasse 1**
**D-8100 Garmisch-Partenkirchen (DE)**

(74) Vertreter: **Mann, Volker, Dr. et al**
**c/o Bayer AG Konzernverwaltung RP Patentabteilung**
**D-5090 Leverkusen 1, Bayerwerk (DE)**

**EP 0 132 690 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft thermoplastisch verarbeitbare Kunststoffe oder Prepolymere enthaltend nichtsteroidale Antiphlogistika sowie die Herstellung solcher Thermoplasten und ihre Verwendung bei der Bekämpfung von Entzündungen.

Für viele medizinische Einsatzgebiete, beispielsweise Kanülen, Katheter, Schläuche, künstliche Blutgefäße, Implantate, Herzklappen, Folien usw. werden Kunststoffe verlangt, die außer ihren spezifischen mechanischen Eigenschaften auch gute Blut- und Gewebeverträglichkeit besitzen. Von besonderem Vorteil können hier lokal begrenzte antiphlogistische Wirkungen sein.

Antiphlogistika werden bisher bei Mensch und Tier oral, rektal oder cutan appliziert.

Bei der Infusionstherapie über Kanülen aus Kunststoff bzw. plastischem Material treten bei längerer Verweildauer vielfach thrombotische und/oder phlebitische Reaktionen der kanülierten Vene auf. Nach A. Fassolt, Schweiz. Rundschau Med. (PRAXIS), 68, 1682-1686 (1979), beträgt die mittlere Häufigkeit venöser Reaktionen bei 4-tägiger Implantationsdauer bereits 50 %. Nach Trans, ophthal. Soc. U.K. 101, 84 bis 86, 1981 können z. B. Nylon und Polypropylen im Humanserum chemotaktisch wirken.

Ferner sind antiinflammatorische und analgetische Pflaster bekannt, die z. B. eine Indomethacinenthaltende copolymerisierte Schicht aufweisen (Nitto Electric Ind. Co., Ltd., Jap. Pat. 126269 vom 29.9.79) oder den Wirkstoff im Kleber enthalten (Jpn. Kokai Tokkyo Koho 81, 20514, 26.2.1981, Appl. 79/96427, 27.7.79).

Aus der GB-A-2 086 224 ist bekannt, in ein Haftmittel auf Polyacrylatbasis Indometacin einzuarbeiten, indem man das Polymer löst, das Indometacin zu der Lösung gibt und dann das Lösungsmittel abdestilliert. Da sich der Wirkstoff nur unzureichend in dem Lösungsmittel löst, erreicht man eine unbefriedigende Verteilung des Wirkstoffs in Polymeren.

In der FR-A-2 372 623 wird beschrieben, Fungizide pulverförmig in ein Polymer (z. B. PVC) einzuarbeiten, wobei für diesen Vorgang Weichmacher, Stabilisatoren und Schmiermittel erforderlich sind (z. B. Beispiel 1). Extruder werden nicht eingesetzt. Bei diesem Verfahren erhält man Polymere, bei denen sich der Wirkstoff an der Oberfläche anreichert.

Die vorbeschriebenen Materialien oder Verfahren sind zur Herstellung medizinischer Bedarfsartikel nur bedingt geeignet.

Eine Aufgabe der vorliegenden Erfindung war es, wirkstoffhaltige thermoplastische Materialien bereitzustellen, die mit den in der Kunststoffindustrie üblichen Verfahren verarbeitet werden können und ohne weitere Nachbehandlung bioverträgliche und antiphlogistische Eigenschaften aufweisen.

Es wurden Antiphlogistika enthaltende thermoplastische Kunststoffe für Kanülen, Katheter, Schläuche, künstliche Blutgefäße, Implantate, Herzklappen und Folien, herstellbar aus thermoplastischen Kunststoffen aus der Gruppe der Polyolefine, Styrolopolymerisate, Polyvinylhalogenide, Polycarbonate, Polyester, Polyamide, Polyphenyloxide, Polyacetale, Celluloseester oder Polyurethane und Copolymerisate der vorgenannten Polymeren und Antiphlogistika der Formel

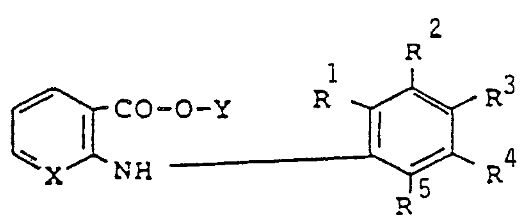

wobei

$R^1$ bis $R^5$ für Wasserstoff, Halogen, niederes Alkyl, substituiertes Alkyl,

X für N oder CH und

Y für Wasserstoff, Metallionen, Alkyl sowie substituiertes Alkyl steht,

und/oder der Formel

$$Ar-(-\overset{O}{\overset{\|}{C}}-)_p-(\overset{R}{\overset{|}{C}}H)_n-(CH_2)_m-COOH$$

wobei

R für Wasserstoff, niedriges Alkyl, substituiertes Alkyl,

Ar für Aryl, Heteroaryl, substituiertes Aryl, substituiertes Heteroaryl steht, und

n + m eine ganze Zahl bedeuten und den Wert Null, 1 oder 2 besitzen und

p' Null oder 1 bedeutet,

mit der Bedingung, daß Ar nicht Aryl oder Heteroaryl bedeutet, wenn m + n und p den Wert Null besitzen,

sowie deren Ester oder Amide, und/oder der Formel

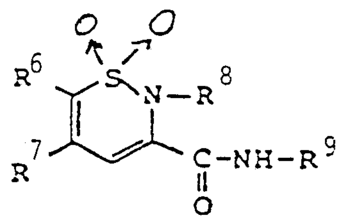

wobei

$R^6$, $R^7$ zusammen einen Aryl- oder Heteroarylring mit einem N-Atom bilden,

$R^8$ eine niedere Alkylgruppe mit 1 bis 4 C-Atomen,

$R^9$ einen Heteroarylrest mit mindestens einem Heteroatom darstellen,

wobei man in einem Temperaturbereich, bei dem der thermoplastische Kunststoff plastifiziert und das Antiphlogistikum in flüssiger Form vorliegt, die Komponenten in einem Extruder mischt und dann homogenisiert, gefunden.

Überraschenderweise ist es möglich, antiphlogistisch wirksame Substanzen in Kunststoffe zu inkorporieren, welche zur Herstellung von Kathedern, Kanülen etc. geeignet sind. Die Wirkstoffe sind ohne Zersetzung und mit guter content uniformity inkorporierbar. Aus den entsprechenden Präparaten werden die Wirkstoffe in vivo über einen mehrtägigen Zeitraum freigesetzt. Hierbei besitzen die Verumkatheder, im Vergleich zu Placebokathedern (ohne Antiphlogistikum), eine bessere Venenverträglichkeit.

Thermoplastisch verarbeitete Kunststoffe — kurz Thermoplasten — sind organische polymere Massen, die verformbare Schmelzen bilden und nach dem Erstarren der Schmelze unverändert sind. Sie können nach verschiedenen bekannten Methoden z. B. durch Extrudieren, Spritzgießen, Pressen, Tiefziehen zu beliebigen Formkörpern verarbeitet werden.

Beispiele für Thermoplasten im Sinne der Erfindung sind : Polymerisate wie Polyolefine (z. B. Polyethylen, Polypropylen, fluorierte Ethylen/Propylen-Copolymerisate, Ethylen/Propylen-Copolymerisate mit thermoelastischen Eigenschaften (EPM Polymere) Ethylen/Acrylsäure-Copolymerisate und Terpolymerisate aus Ethylen, Propylen und geringen Mengen nicht-konjugierter Diene mit thermoplastischen Eigenschaften (EPDM Rubber), Styrolpolymerisate (z. B. Polymethylmethacrylat) ; Polyvinylcyanide (z. B. Polyacrylnitril) ; Polyvinylhalogenide (z. B. Polyvinylchlorid) und eine große Zahl von Mischpolymerisaten aus den oben angegebenen Produkten zugrunde liegenden Monomeren z. B. Copolymerisate von Styrol und Acrylnitril und gegebenenfalls verseifte Copolymerisate von Ethylen und Vinylacetat. Weitere Beispiele sind Polykondensate wie Polycarbonate (z. B. Bisphenol-A-Polycarbonat) ; Polyester (z. B. Polyethylenterephthalat) ; Polyamide (z. B. Polycaprolactam) ; Polyphenylenoxid, Polyacetale (z. B. Polyoximethylen) ; Celluloseester (z. B. Cellulosepropionat) und Polyurethane soweit sie thermoplastische Eigenschaften haben. Für nähere Einzelheiten siehe Kunststoff Taschenbuch 19. Auflage, Seite 207-376, Carl-Hanser-Verlag (1974).

Es sind selbstverständlich auch Thermoplasten geeignet, die nach der Verformung zu Formkörpern noch nachgehärtet werden müssen, oben als Prepolymere bezeichnet.

Nichtsteroidale Antiphlogistika im Sinne der vorliegenden Erfindung sind eines oder mehrere Antiphlogistika der allgemeinen Formeln I und/oder II.

Antiphlogistika der allgemeinen Formel I besitzen die folgende Struktur :

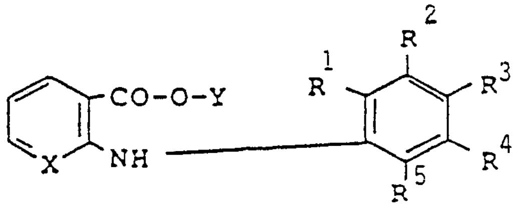

(I)

wobei

$R^1$-$R^5$ Wasserstoff, Halogen, niederes Alkyl, substituiertes Alkyl,

X N oder CH und

Y Wasserstoff, Metallionen, Alkyl oder substituiertes Alkyl,

bedeutet.

Halogen bedeutet Fluor, Chlor, Brom, vorzugsweise Chlor und/oder Brom. Niederes Alkyl ist bevorzugt Alkyl mit 1-6 C-Atomen, besonders bevorzugt 1-4 C-Atomen, substituiertes Alkyl für $R_1$-$R_5$ bedeutet bevorzugt Trihalogenalkyl, besonders bevorzugt Trifluoromethyl. Unter Metallionen sind die Ionen der Alkalimetalle, Erdalkalimetalle und des Aluminiums zu verstehen. Substituiertes Alkyl für Y

bedeutet bevorzugt Alkoxy, Alkoxyalkyl, Hydroxyalkyl, Hydroxyalkoxyalkyl, Trihalogenalkyl, wobei die Anzahl der C-Atome 1 bis 6 beträgt und die Alkylkette gerade oder verzweigt sein kann.

Vorzugsweise werden Antiphlogistika der allgemeinen Formel I eingesetzt, in welcher
$R^3$ und $R^4$ Wasserstoff
X Stickstoff oder eine CH-Gruppe,
Y Wasserstoff, Metallionen, Alkyl oder substituiertes Alkyl und
$R^1$, $R^2$ und $R^5$ Wasserstoff, Halogen, niederes Alkyl oder substituiertes Alkyl bedeuten.

Besonders bevorzugt sind Antiphlogistika der allgemeinen Formel I, in welcher
X für eine CH-Gruppe steht und
$R^1$, $R^2$ und $R^5$ Methyl, Trifluormethyl oder Chlor bedeuten.

Ganz besonders bevorzugt sind die folgenden Antiphlogistika :

1. N-(α,α,α-Trifluor-m-tolyl)-anthranilsäure
= Flufenaminsäure

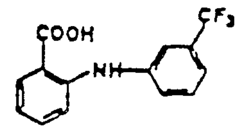

2. N-(2,3-Xylyl)-anthranilsäure

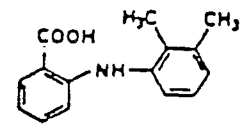

3. N-(2,6-Dichlor-m-tolyl)-anthranilsäure

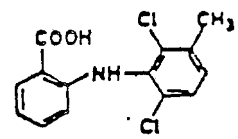

4. N-(3-Chlor-o-tolyl)-anthranilsäure

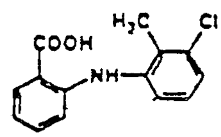

5. N-(2,3-Dichlorphenyl)-anthranilsäure

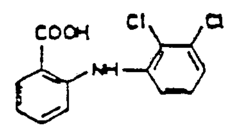

6. N-(α,α,α- Trifluor-m-tolyl)-nicotinsäure
= Nifluminsäure

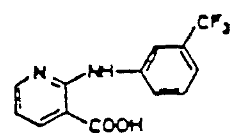

7. N-(2,3-Xylidino)-nicotinsäure

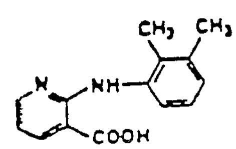

8. 2-(2-Methyl-3-trifluormethylanilino)-nicotinsäure

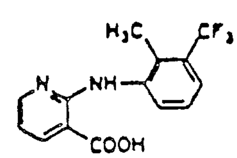

9. N-(3-Chlor-o-tolyl)-nicotinsäure

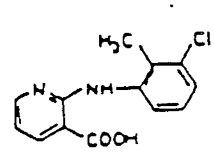

10. 2-(2,6-Xylidino)-nicotinsäure

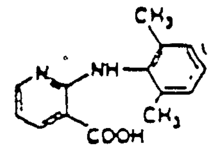

und deren Ester, besonders

11. 2-(2-Hydroxyethoxy)-ethyl-N-(α,α,α-trifluor-m-tolyl)-anthranilat
= Etofenamat

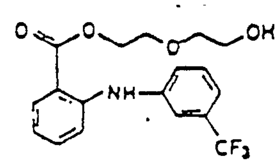

12. Ethoxymethyl-N-(2,6-dichloro-m-tolyl)-anthranilat

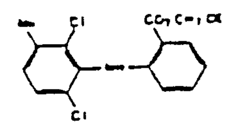

13. Methyl-, Ethyl-, i./n-Propyl-, Butyl-N-(α,α,α-trifluor-m-tolyl)-anthranilat, z. B.

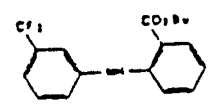

Nichtsteroidale Antiphlogistika im Sinne der vorliegenden Erfindung sind ferner Antiphlogistika der allgemeinen Formel II mit der Struktur :

$$Ar-(-\overset{O}{\overset{\|}{C}}-)_p-(\overset{R}{\overset{|}{C}H})_n-(CH_2)_m-COOH \qquad (II)$$

in welcher
R Wasserstoff, niederes Alkyl, substituiertes Alkyl,
Ar Aryl, Heteroaryl, substituiertes Aryl, substituiertes Heteroaryl und
n + m eine ganze Zahl bedeuten und den Wert Null, 1 oder 2 besitzen,
p Null oder 1 bedeutet,
mit der Bedingung, daß Ar nicht Aryl oder Heteroaryl bedeutet, wenn n und m und p den Wert Null besitzen, sowie deren Ester oder Amide.

Vorzugsweise bedeuten niederes Alkyl R Reste mit 1-6 C-Atomen, bevorzugt 1-4 C-Atomen, substituiertes Alkyl Alkoxyalkyl oder Trihalogenalkyl ; Aryl bzw. Heteroaryl Phenyl, Naphthyl, Thiophenyl, Pyrolyl, Indenyl, Indolyl, Benzthiazinyl, Phenothiazinyl.

Substituenten für Aryl bzw. Heteroaryl sind Alkyl, bevorzugt grad- und verzweigtkettiges Alkyl mit bis zu 6 C-Atomen, Alkoxy, Oxalkyl, Acyl, Hydroxyl, Acetoxy, Benzoyl, substituiertes Benzoyl, Phenyl, substituiertes Phenyl, Phenoxy, Halogen, Phenylalkenyl, Phenylalkyl.

Die Ester sind Alkylester mit 1-6 C-Atomen, bevorzugt 1-4 C-Atomen, besonders bevorzugt Methyl, Ethyl, i- und n-Propyl, substituiertes Alkyl, z. B. β-Hydroxyethyl, Ester mit Glycyolsäure. Die Amide können in der Gruppierung $-CO-NH_2$ an Stelle eines oder beider Amid-Wasserstoffe auch niedere Alkyle bzw. substituiertes Alkyle enthalten.

Besonders bevorzugt sind die folgenden Antiphlogistika der allgemeinen Formel II :

14. 2-Hydroxybenzoesäure

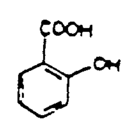

15. 2-Acetoxybenzoesäure

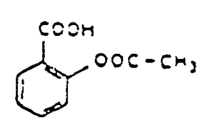

16. 2′,4′-Difluor-4-hydroxy-3-biphenylcarbonsäure

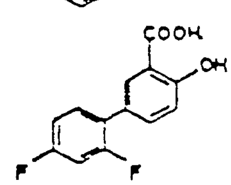

17. 2-Hydroxybenzamid

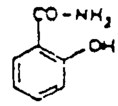

18. [2-(Aminocarbonyl)phenoxyl]-essigsäure

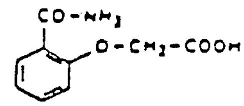

19. 4-Allyloxy-3-chlorphenyl-essigsäure
= Alclofenac

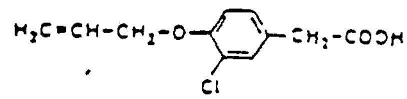

20. 2-[(2,6-Dichlorphenyl)amino]-phenylessigsäure
= Diclofenac

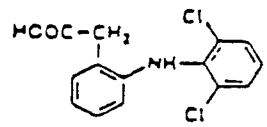

21. 10-Methyl-phenothiazin-2-ylessigsäure
= Metiazinsäure

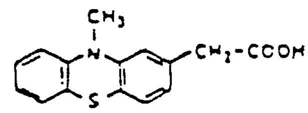

22. 1-Methyl-5-(p-toluoyl)-pyrrol 2-yl-essigsäure

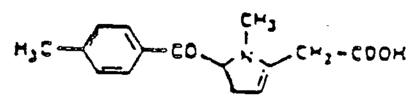

23. D-2-(6-Methoxy-2-naphthyl)-propionsäure
= Naproxen

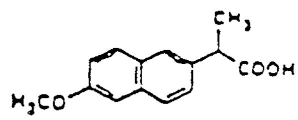

24. 2-(p-Isobutylphenyl)-propionsäure

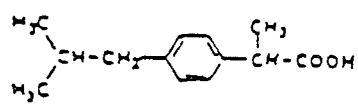

25. 2-(3-Phenoxyphenyl)-propionsäure

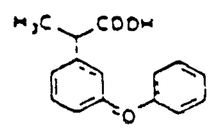

26. 2-(m-Benzoylphenyl)-propionsäure
= Ketoprofen

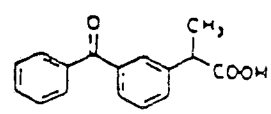

27. 2-[4-(1-Oxo-2-isoindolinyl)-phenyl]-propionsäure
= Indoprofen

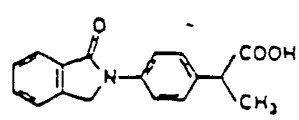

28. 2-(2-Fluorbiphenyl-4-yl)-propionsäure

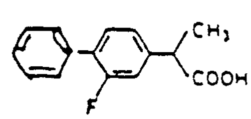

29. 3-(4-Biphenylylcarbonyl)-propionsäure

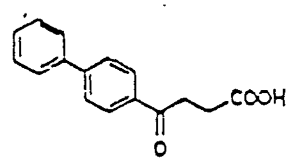

30. 2-(5-Benzoyl-2-thienyl)-propionsäure

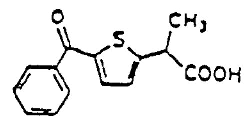

31. 1-(p-Chlorbenzoyl)-5-methoxy-2-methylindol-3-essigsäure
= Indometacin

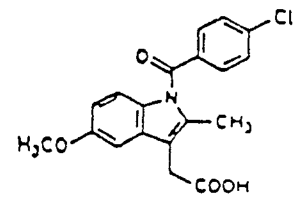

32. [1-(p-Chlorbenzoyl)-5-methoxy-2-methylindol-3-acetoxy]essigsäure
= Acemetacin

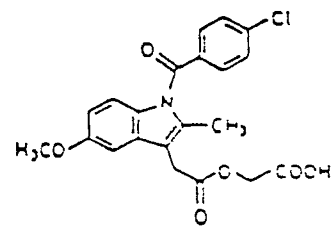

33. (Z)-5-Fluor-2-methyl-1-{[(4-methyl=sulfinyl)phenyl]methylen}-1H-inden-3-essigsäure

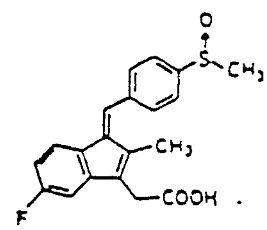

34. 4-Butyl-1,2-diphenyl-3,5-pyrazolidin-dion
= Phenylbutazon

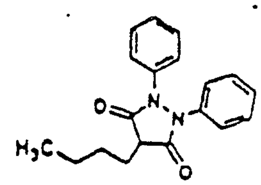

35. 4-Prenyl-1,2-diphenyl-pyrazolidin-3,5-dion
= Feprazon

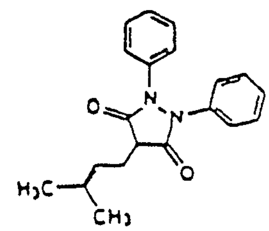

sowie deren Alkylester und substituierte Alkylester.

Bevorzugt sind ferner die folgenden Antiphlogistika der allgemeinen Formel III :

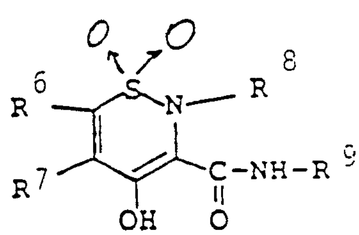

(III)

in welcher

$R^6$ und $R^7$ zusammen einen Aryl- oder Heteroarylring mit einem Stickstoffatom bilden,

$R^8$ eine niedere Alkylgruppe mit 1-4 C Atomen,

$R^9$ einen Heteroarylrest mit mindestens einem Heteroatom darstellen.

Besonders bevorzugt sind die folgenden Antiphlogistika der allgemeinen Formel III :

36. 4-Hydroxy-2-methyl-N-2-thiazolyl-2H-1,2-benzothiazin-3-carboxamid-1,1-dioxid 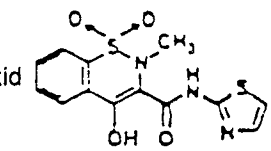

37. 4-Hydroxy-2-methyl-N-2-pyridinyl-2H-1,2-benzothiazin-3-carboxamid-1,1-dioxid 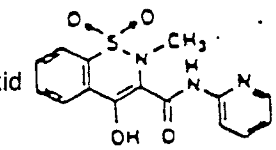

Die vorstehend genannten Antiphlogistika der allgemeinen Formel I und II können sowohl einzeln als auch zu mehreren in die Thermoplasten eingearbeitet werden.

Die Antiphlogistika können in einer Menge von 1-30 Gew.-%, vorzugsweise 2-20 Gew.-% in die Thermoplasten eingearbeitet werden.

Hierzu kann man sich in der Kunststoffcompoundierung üblicher Methoden bedienen. Beispielsweise kann man das Antiphlogistikum in fester oder in flüssiger Form mit den als Pulver oder Granulat vorliegenden Thermoplasten mischen und die Mischung in einem Extruder aufschmelzen, homogenisieren und das Extrudat wieder granulieren. Man kann auch das Antiphlogistikum, ebenfalls fest oder flüssig, auf dem Walzenstuhl in das Polymerisat einarbeiten. Das dabei entstehende Walzfell kann anschließend granuliert werden. Es ist auch möglich, das Antiphlogistikum durch Kneten in einer üblichen Knetvorrichtung einzuarbeiten. Die Arbeitstemperaturen müssen für jeden einzelnen Fall so gewählt werden, daß der thermoplastische Kunststoff sich plastifizieren läßt, aber die Zersetzungstemperatur des einzuarbeitenden Wirkstoffs nicht erreicht wird. Es hat sich herausgestellt, daß bevorzugt für gute Homogenität des Produktes, der Wirkstoff in flüssiger Form vorliegen soll, d. h. geschmolzen oder gegebenenfalls gelöst in einem geeigneten Lösungsmittel.

Bevorzugt arbeitet man Verbindungen mit niedrigem Schmelzpunkt und guter Thermostabilität ein, z. B. Alclofenac bei 91-170 °C, Ketoprofen bei 94-180 °C, Phenylbutazon bei 105-180 °C, Etofenamat bis 180 °C, Diclofenac(säure) bei 156-176 °C. Weiter besonders geeignet sind Verbindungen mit Schmelzpunkten bis 160° und guter Thermostabilität, z. B. Acemetacin (Einarbeitung 150-190 °C, Flufenaminsäure 133-180 °C, Indometacin 155-180 °C, Naproxen 154-180 °C, Feprazon 153-180 °C und Metiazinsäure 146-160 °C).

Weiter besonders geeignet sind Verbindungen mit hohem Schmelzpunkt, die sich nicht zersetzen, z. B. Nifluminsäure (Einarbeitung bei 204-220 °C), Sulindac (189-210 °C) und Indoprofen (213-220 °C).

Die in vivo Liberation der Antiphlogistika aus Kunststoffkathetern konnte tierexperimentell belegt werden. Die Versuche wurden mit männlichen Ratten [Stamm : Bor : WISW (SPF-Cpb), Gewicht um 200 g] durchgeführt. Polyethylenkatheter mit Verbindung 11 wurden Ratten in eine Vena femoralis in Höhe der Leistenbeuge vorgeschoben, der Katheter durch eine Außennaht rund um das Gefäß, die nicht zu einer Ligatur führte, fixiert. Das Katheterende wurde verschweißt. Die Applikationsdauer betrug 1, 7, bzw. 10 Tage. Danach wurde der Gehalt an Verbindung 11, im Vergleich zu nicht implantierten 11-haltigen Kathetern ermittelt. Die liberierte Menge an Wirkstoff ergibt sich aus der Differenz des Gehaltes vor Inkorporation und nach unterschiedlich langer Verweildauer. Bei Ratten wurde bis zu 59,2 ± 4,9 % der Verbindung 11 in vivo liberiert.

Auch bei Kaninchen (Installation des Katheters in die Ohrvene) wurde eine, mit der Verweildauer ansteigende Liberation bis zu ca. 75 % (10. Tag) festgestellt.

Die Gewebeverträglichkeit wurde durch Vergleich von Verum- und Placebo-Kathetern (mit und ohne Antiphlogistikum) jeweils am gleichen Tier geprüft.

Die Schutzwirkung der Verbindung 11, welche in PE-Katheter inkorporiert war, konnte an Kaninchen (männliche Weiße Neuseeländer ; Gewicht 2,3-4,1 kg) demonstriert werden. Den Tieren wurden Verum- und Placebokatheter (mit bzw. ohne 11) in Ohrvenen inkorporiert, fixiert und 7 Tage in den Venen belassen. Die Abbildungen zeigen unterschiedliche Venenquerschnitte.

In Abb. 1 ist ein Venenquerschnitt nach 7-tägiger Implantation eines Placebo-Katheters, in Abb. 2 nach 7-tägiger Implantation eines Verumkatheters dargestellt :

(Siehe Abbildung 1 Seite 9 f.)

Abbildung 1

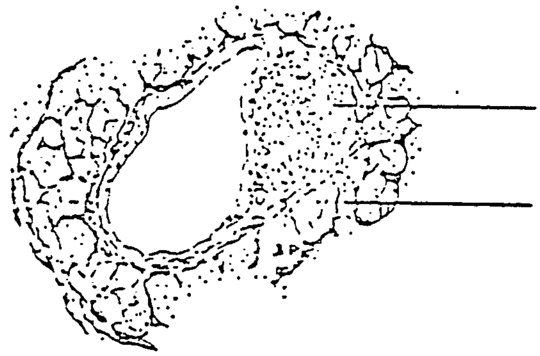


Ausgedehnte entzündliche Reaktion in der Ohrvene des Kaninchens am 7. Tag nach der Implantation eines PE-Katheters.

Abbildung 2

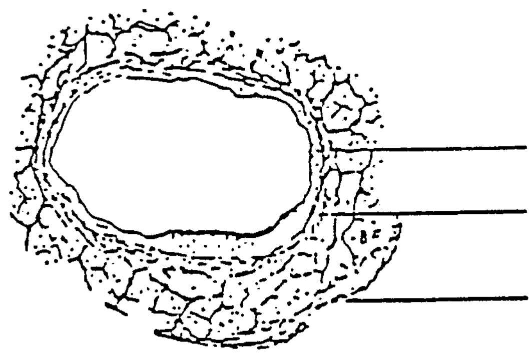


Die Implantation eines 11-haltigen PE-Katheters in die Ohrvene des Kaninchens ruft nahezu keine Entzündungsreaktion hervor, 7. Tag nach Implantation.

Es ist deutlich zu erkennen, daß unter dem Einfluß des in vivo liberierten Wirkstoffes eine entzündliche Reaktion im Vergleich zum Placebo weitgehend verhindert wird.

Die in vivo Liberation der Verbindungen 1 und 11, welche in Mengen von 10 % in Polyethylen-Katheter inkorporiert waren, wurde an Ratten [männliche Tiere, Stamm : BOR ; WISW (SpF-Cpb), Gewicht um 200 mg], denen die Katheter über eine « Braun'sche Kanüle » in den Intraperitonealraum gegeben wurden und welche dort 3 bzw. 7 Tage lang verblieben, nachgewiesen. Die Liberation wurde durch Ermittlung des Restgehaltes an 1 bzw. 11 im Katheter nach mehrtägiger Applikation, im Vergleich zum nicht implantierten Verumkatheter bestimmt.

| Wirkstoff | Applikation (d) | % in vivo Liberation |
|---|---|---|
| 1 | 3 | 57,7 ± 3,7 |
| | 7 | 78,9 ± 3,4 |
| 11 | 3 | 75,0 ± 1,7 |
| | 7 | 86,3 ± 4,8 |
| 34 | 3 | 51,4 + 6,0 |
| | 7 | 79,8 + 2,2 |

In vitro läßt sich in Ethylen-Propylen-Copolymer inkorporiertes Etofenamat mit Aceton quantitativ isolieren, während Etofenamat aus Polyurethan bevorzugt mit Ethanol extrahiert wird (Siedehitze, unter Rühren und Rückfluß). Die in vitro-Liberation erfolgt aus Polyurethan schneller als aus dem Ethylen-Propylen-Copolymer. Es treten keine Neben- oder Zersetzungsprodukte auf, die content uniformity ist gut.

Beispiel 1

1 000 g Polyethylen (Baylon 22H764) wurden auf einem Troester Labormischwalzwerk 40 Minuten bei 130 °C unter portionsweiser Zugabe von insgesamt 100 g Etofenamat gewalzt, anschließend granuliert. Es resultierte ein gleichmäßig opakes Granulat, das sich zu homogenen Preßplatten von 0,3 bis 2 mm Dicke weiterverarbeiten ließ. Der Wirkstoff war chemisch unverändert und homogen im Kunststoff verteilt.

Beispiel 2

900 g Polyethylen (Baylon 22H764) und 100 g Etofenamat wurden bei 160 °C zu einem opaken Walzfell verarbeitet und anschließend granuliert. Das Granulat wurde dann auf einem Laborextruder bei Massetemperaturen von 160-170 °C zu Schläuchen von 1-3 mm Durchmesser verarbeitet. Diese Schläuche enthielten 10 % unverändertes Etofenamat in gleichmäßiger Verteilung und zeigten ein opakes Aussehen sowie eine « fettig » erscheinende Oberfläche. Labor- und Tierversuche bewiesen eine deutliche Wirkstoffabgabe und signifikant positive physiologische Wirkungen (s. oben).

Beispiel 3

1 000 g Polyethylen (Baylon 19N430) und 100 g Etofenamat (Verbindung 11) wurden bei 90-95 °C zu einem Walzfell verarbeitet, anschließend granuliert und zu homogenen Platten gepreßt.

Beispiel 4

400 g Ethylen/Propylen-Copolymer (Levaflex EP 281) und 83,6 g Etofenamat wurden bei 170 °C zu einem Walzfell verarbeitet, anschließend granuliert und zu homogenen, sehr flexiblen, anschmiegsamen, 0,5 mm dicken Platten gepreßt.

Beispiel 5

1 000 g Ethylen/Vinylalkohol-Copolymer (Levasint S 31 natur) und 100 g Etofenamat wurden bei 90 °C zu einem Walzfell verarbeitet, anschließend granuliert und zu homogenen Platten gepreßt.

Beispiel 6

500 g Polyethylen (Baylon 19N430) und 52,5 g Indometacin wurden auf einem Mischwalzwerk « Schwabenthan 200 » bei 155 °C in 10 Minuten zu einem weißen Walzfell verarbeitet, anschließend granuliert und bei 150 °C zu Schläuchen von 1-3 mm Durchmesser extrudiert. Das Material zeigte gute content uniformity, deutliche Wirkstoffreisetzung in vivo und positive physiologische Wirkung (s. oben).

Beispiel 7

500 g Polyethylen (Baylon 19N430) und 53,1 g Flufenaminsäure wurde bei 155 °C zu einem bernsteinfarbenen Walzfell verarbeitet, anschließend granuliert und bei 150 °C zu dünnen Schläuchen extrudiert. Das Material zeigte gute content uniformity, deutliche Wirkstoffliberation in vivo und positive physiologische Wirkung (s. oben).

Beispiel 8

500 g Polyethylen (Baylon 19N430) und 51,9 g Acemetacin wurden bei 155 °C zu einem gelben Walzfell verarbeitet, anschließend granuliert und zu 0,3 mm dicken Platten gepreßt. Der Wirkstoff war chemisch unverändert und homogen im Kunststoff verteilt. Wirkstoffliberation und pos. physiologische Wirkung wurde nachgewiesen (s. oben).

Beispiel 9

500 g Polyethylen (Baylon 19N430) und 50 g Phenylbutazon wurden bei 140 °C zu einem Walzfell verarbeitet, anschließend granuliert und bei 150 °C zu dünnen Schläuchen extrudiert. Das Material zeigte gute content uniformity, deutliche Wirkstoffliberation in vivo und positive physiologische Wirkung (s. oben).

Beispiel 10

500 g thermoplastisches Polyurethan (für medizinische Bedarfsartikel geeignetes PU 786 der Bayer AG) und 50 g Etofenamat wurden bei 170 °C zu einem Walzfell verarbeitet, anschließend granuliert und zu 0,3 mm dicken Platten gepreßt. Es resultierte ein nahezu transparentes, hochflexibles Material hoher Reißdehnung (> 600 %). Der Wirkstoff war homogen im Kunststoff verteilt und wurde chemisch unverändert wieder freigesetzt.

Beispiel 11

500 g thermoplastisches Polyurethan auf Basis MDI/Polyether/Polyester (Desmopan 786 der Bayer AG) und 50 g Piroxicam wurden bei 210 °C zu einem Walzfell verarbeitet, anschließend granuliert und zu hochflexiblen, 0,3 mm dicken Platten gepreßt. Der Wirkstoff war homogen im Kunststoff verteilt und wurde chemisch unverändert freigesetzt.

Beispiel 12

500 g Polyethylen (PE 22H768 der Bayer AG) und 50 g Diclofenac (Säure) wurden bei 170-175 °C zu einem Walzfell verarbeitet, anschließend granuliert und zu 0,3 mm dicken Platten gepreßt. Der Wirkstoff war homogen im Kunststoff verteilt und wurde chemisch unverändert wieder freigesetzt.

**Patentansprüche**

1. Antiphlogistika enthaltende thermoplastische Kunststoffe für Kanülen, Katheter, Schläuche, künstliche Blutgefäße, Implantate, Herzklappen und Folien, herstellbar aus thermoplastischen Kunststoffen aus der Gruppe der Polyolefine, Styrolopolymerisate, Polyvinylhalogenide, Polycarbonate, Polyester, Polyamide, Polyphenylenoxide, Polyacetale, Celluloseester oder Polyurethane und Copolymerisate der vorgenannten Polymeren und Antiphlogistika der Formel

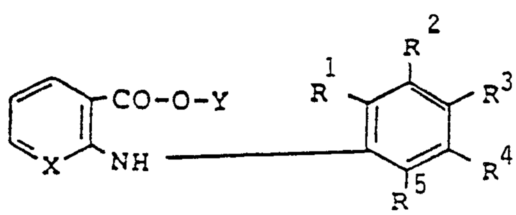

wobei
$R^1$ bis $R^5$ für Wasserstoff, Halogen, niederes Alkyl, substituiertes Alkyl,
X für N oder CH und
Y für Wasserstoff, Metallionen, Alkyl sowie substituiertes Alkyl steht,
und/oder der Formel

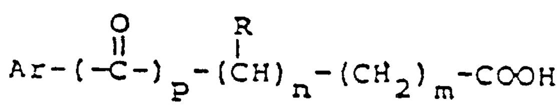

wobei
R für Wasserstoff, niedriges Alkyl, substituiertes Alkyl,
Ar für Aryl, Heteroaryl, substituiertes Aryl, substituiertes Heteroaryl steht, und
n + m eine ganze Zahl bedeuten und den Wert Null, 1 oder 2 besitzen und
p Null oder 1 bedeutet,
mit der Bedingung, daß Ar nicht Aryl oder Heteroaryl bedeutet, wenn m + n und p den Wert Null besitzen, sowie deren Ester oder Amide, und/oder der Formel

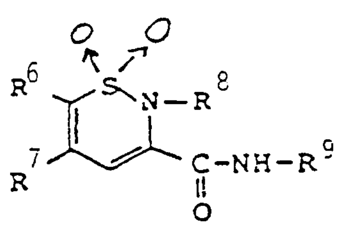

wobei

$R^6$, $R^7$ zusammen einen Aryl- oder Heteroarylring mit einem N-Atom bilden,

$R^8$ eine niedere Alkylgruppe mit 1-4 C-Atomen,

$R^9$ einen Heteroarylrest mit mindestens einem Heteroatom darstellen,

wobei man in einem Temperaturbereich, bei dem der thermoplastische Kunststoff plastifiziert und das Antiphlogistikum in flüssiger Form vorliegt, die Komponenten in einem Extruder gemischt und dann homogenisiert hat.

2. Verfahren zur Herstellung von thermoplastisch verarbeitbaren Kunststoffen, die nichtsteroidale Antiphlogistika enthalten, dadurch gekennzeichnet, daß man Antiphlogistika der Formel

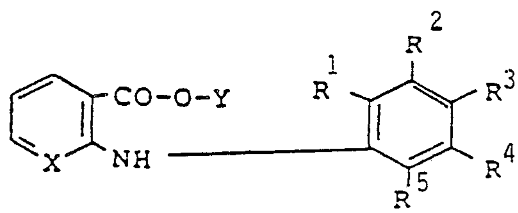

wobei

$R^1$ bis $R^5$ für Wasserstoff, Halogen, niederes Alkyl, substituiertes Alkyl,

X für N oder CH und

Y für Wasserstoff, Metallionen, Alkyl sowie substituiertes Alkyl steht,

und/oder der Formel

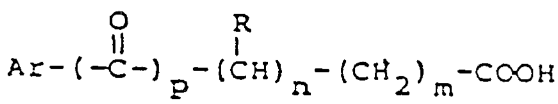

wobei

R für Wasserstoff, niedriges Alkyl, substituiertes Alkyl,

Ar für Aryl, Heteroaryl, substituiertes Aryl, substituiertes Heteroaryl steht, und

n + m eine ganze Zahl bedeuten und den Wert Null, 1 oder 2 besitzen und

p Null oder 1 bedeutet,

mit der Bedingung, daß Ar nicht Aryl oder Heteroaryl bedeutet, wenn m + n und p den Wert Null besitzen, sowie deren Ester oder Amide, und/oder der Formel

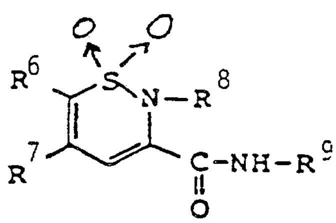

wobei

$R^6$, $R^7$ zusammen einen Aryl- oder Heteroarylring mit einem N-Atom bilden,

$R^8$ eine niedere Alkylgruppe mit 1-4 C-Atomen,

$R^9$ einen Heteroarylrest mit mindestens einem Heteroatom darstellen

in fester oder flüssiger Form mit einem als Pulver oder Granulat vorliegenden Thermoplasten mischt, die Mischung in einem Temperaturbereich, bei dem der Thermoplast plastifiziert und das Antiphlogistikum in flüssiger Form vorliegt, in einem Extruder mischt und dann homogenisiert.

3. Verfahren nach Anspruch 2, dadurch gekennzeichnet, daß man die Homogenisierung in einem Walzenstuhl vornimmt.

4. Verfahren nach Anspruch 2, dadurch gekennzeichnet, daß man die Homogenisierung in einer Knetvorrichtung vornimmt.

5. Verfahren nach den Ansprüchen 2 bis 4, dadurch gekennzeichnet, daß man das Antiphlogistikum in geschmolzener oder gelöster Form in den thermoplastischen Kunststoff einarbeitet.

6. Verfahren nach den Ansprüchen 2 bis 5, dadurch gekennzeichnet, daß man Alclofenac bei Temperaturen von 91 bis 170 °C, Ketoprofen bei Temperaturen von 94 bis 180 °C, Phenylbutazon bei Temperaturen von 105 bis 180 °C, Etofenamat bei Temperaturen bis 180 °C, Diclofenac (Säure bei Temperaturen von 156 bis 176 °C, Acemetacin bei Temperaturen von 150 bis 190 °C, Flufenaminsäure bei Temperaturen von 133 bis 180 °C, Indometacin bei Temperaturen von 155 bis 180 °C, Naproxen bei

Temperaturen von 154 bis 180 °C, Feprazon bei Temperaturen von 146 bis 160 °C. Nifluminsäure bei Temperaturen von 204 bis 220 °C, Sulindac bei Temperaturen von 189 bis 210 °C oder Indoprofen bei Temperaturen von 213 bis 220 °C in einen plastifizierten thermoplastischen Kunststoff einarbeitet.

7. Verwendung von Antiphlogistika enthaltenden Kunststoffen nach Anspruch 1 zur Herstellung von Kanülen oder Katheter.

**Claims**

1. Thermoplastics containing antiinflammatory agents for cannulas, catheters, tubes, artificial blood vessels, implants, heart valves and films, which can be produced from thermoplastics from the group comprising polyolefines, styrene polymers, polyvinyl halides, polycarbonates, polyesters, polyamides, polyphenylene oxides, polyacetals, cellulose esters or polyurethanes and copolymers of the abovementioned polymers and antiinflammatory agents of the formula

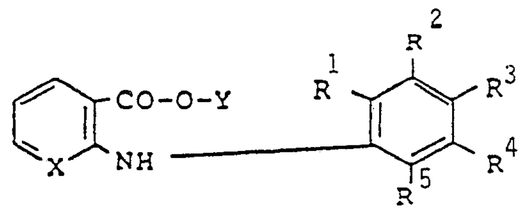

wherein

$R^1$ to $R^5$ represent hydrogen, halogen, lower alkyl or substituted alkyl,

X represents N or CH and

Y represents hydrogen, metal ions, alkyl or substituted alkyl,

and/or of the formula

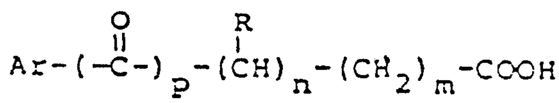

wherein

R represents hydrogen, lower alkyl or substituted alkyl,

Ar represents aryl, heteroaryl, substituted aryl or substituted heteroaryl and

n + m denote an integer and have the value zero, 1 or 2 and

p denotes zero or 1,

with the proviso that Ar does not denote aryl or heteroaryl if m + n and p have the value zero, and esters or amides thereof, and/or of the formula

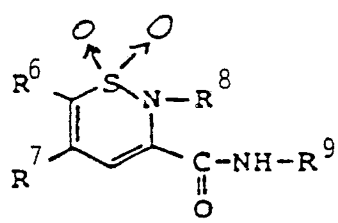

wherein

$R^6$ and $R^7$ together form an aryl or heteroaryl ring with an N atom,

$R^8$ represents a lower alkyl group with 1-4 C atoms and

$R^9$ represents a heteroaryl radical with at least one heteroatom,

wherein the components have been mixed in an extruder and then homogenized, in a temperature range at which the thermoplastic plasticizes and the antiinflammatory agent is in liquid form.

2. Process for the preparation of thermoplastically processable plastics which contain non-steroid antiinflammatory agents, characterized in that antiinflammatory agents of the formula

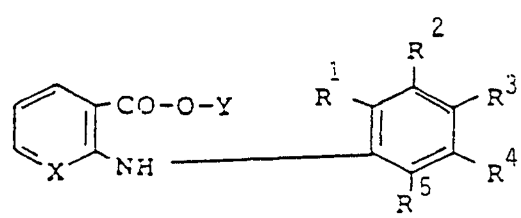

wherein

$R^1$ to $R^5$ represent hydrogen, halogen, lower alkyl or substituted alkyl,

X represents N or CH and

Y represents hydrogen, metal ions, alkyl or substituted alkyl,

and/or of the formula

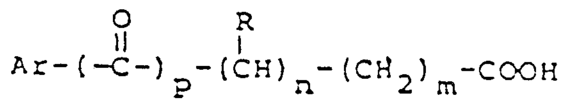

wherein

R represents hydrogen, lower alkyl or substituted alkyl,

Ar represents aryl, heteroaryl, substituted aryl or substituted heteroaryl and

n + m denote an integer and have the value zero, 1 or 2 and

p denotes zero or 1,

with the proviso that Ar does not denote aryl or heteroaryl if m + n and p have the value zero, and esters or amides thereof, and/or of the formula

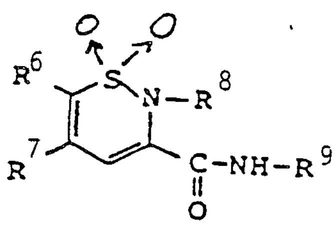

wherein

$R^6$ and $R^7$ together form an aryl or heteroaryl ring with an N atom,

$R^8$ represents a lower alkyl group with 1-4 C atoms and

$R^9$ represents a heteroaryl radical with at least one heteroatom

are mixed in solid or liquid form with a thermoplastic which is in the form of powder or granules, the mixture is mixed in an extruder, in a temperature range at which the thermoplastic plasticizes and the antiinflammatory agent is in liquid form, and the mixture is then homogenized.

3. Process according to Claim 2, characterized in that the homogenization is carried out in a roll mill.

4. Process according to Claim 2, characterized in that homogenization is carried out in a kneading device.

5. Process according to Claims 2 to 4, characterized in that the antiinflammatory agent is incorporated into the thermoplastic in molten or dissolved form.

6. Process according to Claims 2 to 5, characterized in that there is incorporated into a plasticized thermoplastic alclofenac at a temperature of 91 to 170 °C, ketoprofen at a temperature of 94 to 180 °C, phenylbutazone at a temperature of 105 to 180 °C, etofenamate at a temperature of up to 180 °C, diclofenac (acid) at a temperature of 156 to 176 °C, acemetacin at a temperature of 150 to 190 °C, flufenamic acid at a temperature of 133 to 180 °C, indometacin at a temperature of 155 to 180 °C, naproxen at a temperature of 154 to 180 °C, feprazone at a temperature of 146 to 160 °C, niflumic acid at a temperature of 204 to 220 °C, sulindac at a temperature of 189 to 210 °C or indoprofen at a temperature of 213 to 220 °C.

7. Use of thermoplastics containing antiinflammatory agents, according to Claim 1, for the production of cannulas or catheters.

**Revendications**

1. Matière synthétique thermoplastique contenant des anti-inflammatoires pour canules, cathéters, flexibles, vaisseaux sanguins artificiels, implants, valves cardiaques et feuilles pouvant être fabriquée au départ de matières thermoplastiques appartenant au groupe des polyoléfines, des polymères du styrène, des halogénures de polyvinyle, des polycarbonates, des polyesters, des polyamides, des oxydes de polyphénylène, des polyacétals, des esters cellulosiques ou des polyuréthanes et des copolymères des polymères précités et d'anti-inflammatoires répondant à la formule

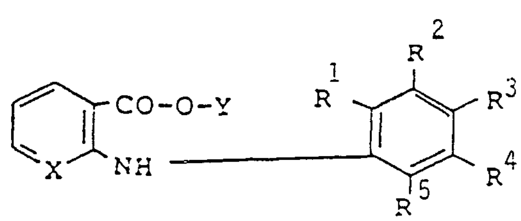

dans laquelle

$R^1$ à $R^5$ représentent de l'hydrogène, un halogène, un alkyle inférieur, un alkyle substitué,

X représente N ou CH et

Y représente de l'hydrogène, des ions métalliques, un alkyle ou un alkyle substitué,

et/ou répondant à la formule

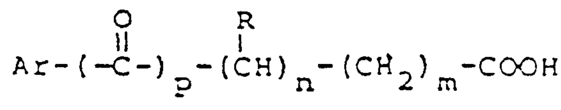

dans laquelle

R représente de l'hydrogène, un alkyle inférieur, un alkyle substitué,

Ar représente un aryle, un hétéroaryle, un aryle substitué, un hétéroaryle substitué, et

n + m représentent un nombre entier et ont pour valeur zéro, 1 ou 2, et

p signifie zéro ou 1,

avec la condition que Ar ne représente pas un aryle ou un hétéroaryle lorsque n + m et p ont pour valeur zéro,

ainsi que leurs esters ou amides, et/ou répondant à la formule

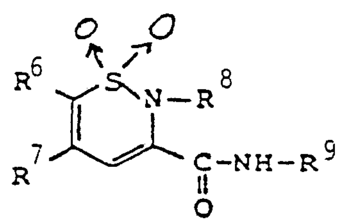

dans laquelle

$R^6$, $R^7$ forment ensemble un anneau arylique ou hétéroarylique avec un atome d'azote,

$R^8$ représente un groupe alkyle inférieur à 1 à 4 atomes de carbone,

$R^9$ représente un radical hétéroaryle avec au moins un hétéro-atome,

en opérant dans une gamme de température où la matière thermoplastique est plastifiée et où l'anti-inflammatoire se trouve à l'état liquide, les composants étant mélangés dans une extrudeuse et homogénéisés ensuite.

2. Procédé de production de matières plastiques pouvant être mises en forme par voie thermoplastique, qui contiennent des anti-inflammatoires non stéroïdaux, caractérisé en ce que l'on mélange des anti-inflammatoires répondant à la formule

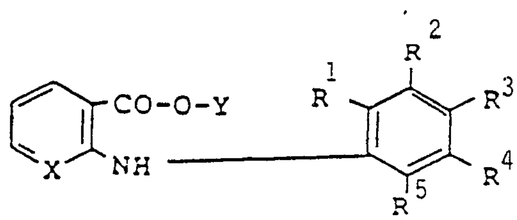

dans laquelle

$R^1$ à $R^5$ représentent de l'hydrogène, un halogène, un alkyle inférieur, un alkyle substitué,

X représente N ou CH, et

Y représente de l'hydrogène, des ions métalliques, un alkyle ou un alkyle substitué,

et/ou répondant à la formule

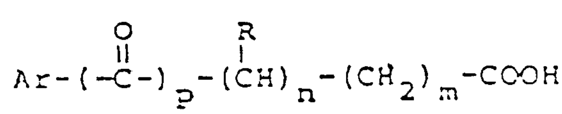

dans laquelle

R représente de l'hydrogène, un alkyle inférieur, un alkyle substitué,

Ar représente un aryle, un hétéroaryle, un aryle substitué, un hétéroaryle substitué, et

n + m représentent un nombre entier et ont pour valeur zéro, 1 ou 2, et

p a pour valeur zéro ou 1,

avec pour condition que Ar ne représente pas un aryle ou un hétéroaryle lorsque n + m et p ont pour valeur zéro, ainsi que leurs esters ou amides, et/ou répondant à la formule

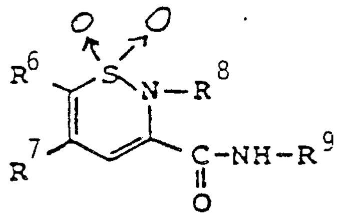

dans laquelle

$R^6$, $R^7$ forment ensemble un anneau arylique ou hétéroarylique à un atome d'azote,

$R^8$ représente un groupe alkyle inférieur à 1-4 atomes de carbone,

$R^9$ représente un radical hétéroarylique avec au moins un hétéro-atome

à l'état solide ou liquide, avec un thermoplaste sous forme de poudre ou de granulés, ce mélange étant plastifié dans une gamme de température où le thermoplaste est plastifié et où l'anti-inflammatoire se trouve à l'état liquide, ce mélange étant mélangé dans une extrudeuse et ensuite homogénéisé.

3. Procédé selon la revendication 2, caractérisé en ce que l'homogénéisation est réalisée dans un mélangeur à rouleaux.

4. Procédé selon la revendication 2, caractérisé en ce que l'homogénéisation est réalisée dans un malaxeur.

5. Procédé selon les revendications 2 à 4, caractérisé en ce que l'on incorpore l'anti-inflammatoire à l'état fondu ou dissous dans la matière thermoplastique.

6. Procédé selon les revendications 2 à 5, caractérisé en ce que l'on introduit dans une matière synthétique thermoplastique plastifiée de l'alclophénac à des températures de 91 à 170 °C, du cétoprophène à des températures de 94 à 180 °C, de la phénylbutazone à des températures de 105 à 180 °C, de l'éthophénamate à des températures jusqu'à 180 °C, du diclophénac (acide) à des températures de 156 à 176 °C, de l'acémétacine à des températures de 150 à 190 °C, de l'acide fluphénaminique à des températures de 133 à 180 °C, de l'indométacine à des températures de 155 à 180 °C, du naproxène à des températures de 154 à 180 °C, de la féprazone à des températures de 146 à 160 °C, de l'acide nilfluminique à des températures de 204 à 220 °C, du sulindac à des températures de 189 à 210 °C ou de l'indoprophène à des températures de 213 à 220 °C.

7. Utilisation de matières plastiques contenant des anti-inflammatoires selon la revendication 1, pour la fabrication de cathéters ou de canules.